(19) Europäisches Patentamt
European Patent Office
Office européen des brevets

(11) EP 3 730 939 A1

(12) **EUROPEAN PATENT APPLICATION**

(43) Date of publication:
**28.10.2020 Bulletin 2020/44**

(51) Int Cl.:
*G01N 33/487* (2006.01)

(21) Application number: **19178617.7**

(22) Date of filing: **06.06.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2019 US 201916395910**

(71) Applicant: **The University of Ottawa**
**Ottawa, Ontario K1N 6N5 (CA)**

(72) Inventors:
- **Briggs, Kyle**
  **Ottawa, Ontario K1N 6N5 (CA)**
- **Charron, Martin**
  **Gatineau, Québec J8R 2I9 (CA)**
- **Tabard-Cossa, Vincent**
  **Ottawa, Ontario K1N 6N5 (CA)**

(74) Representative: **Bittner, Thomas L.**
**Boehmert & Boehmert**
**Anwaltspartnerschaft mbB**
**Pettenkoferstrasse 22**
**80336 München (DE)**

# (54) CALIBRATING NANOPORE CAPTURE RATES USING CONTROLLED COUNTING

(57) Methods for determining molecular concentration using solid-state nanopores are provided. The methods include measuring a current signal and using the signal to identify first and second electrical signatures. A first set is used to determine a first rate of a target having an unknown concentration, and a second set is used to determine a second rate of a control having a known concentration. The first and second rates are each a function of a pore property factor that represents physical properties of the solid-state nanopore and operating conditions. The first rate is further a function of a first property factor that represents physical properties of the target and the unknown concentration. The second rate is further a function of a second property factor that represents physical properties of the control and the known concentration. Ratios of the first rate to the second rate are used to determine the unknown concentration.

110
100
110
r*
106
102
104

FIG. 1

EP 3 730 939 A1

## Description

The present disclosure relates to solid-state nanopores and the precise determination of molecular concentrations by solid-state nanopores using real-time calibration of capture rate measurements, which includes the use of internal calibrators and the controlled counting of single molecules.

## Background

Nanopores are being used as single-molecule sensors to measure concentrations of clinically relevant target analysts, including a range of biological molecules where low abundance biomarkers can herald early onset of many diseases and enable earlier, lower-cost treatments. For example, single-molecule nanopore sensors are enabling the quantification, in real time, of clinically relevant biomarker concentrations, with high sensitivity and in point-of-care format. However, current nanopore-based methods are prone to variability inherent in the capture processes due to uncontrolled sources of variability, including within a single nanopore over time and between different nanopores having nominally identical sizes. Accordingly, it would be desirable to develop methods that improve the precision and accuracy of single-molecule nanopore sensors as a quantitative tool.

Moreover, because DNA is well-suited to the conditions in which such solid-state nanopores optimally function, most nanopore research has focused on DNA as the primary target molecule. However, many clinically relevant targets are proteins. In such instances, operating conditions for solid-state nanopores must be carefully tailored because, for example, proteins are often incompatible with the high-molarity salt concentrations required for high nanopore signal. Further, proteins have transport properties that are complex because of their low and non-uniform charge distribution, as well as their secondary and tertiary structures. In instances when proteins can be directly detected in nanopores, the translocation events are often too fast for the limited time-resolution available to conventional current amplifiers.

In contrast, DNA is able to withstand the higher molar salt concentrations and its high and uniform charge distribution allows for simpler transport properties. As such, several recent studies directed to the detection of proteins and other biomarkers, including small molecules and viruses, have used linear or slightly modified linear DNA as a surrogate label or carrier for the target analyte. Using DNA surrogate labels allow for more robust nanopore signals that are representative of a concentration of the true target analyte, while also permitting multiplexed detection. As such, improved methods for measuring the concentration of DNA would improve the precision and accuracy of concentration measures for a wide range of clinically relevant biomarkers, including specific DNA sequences, miRNA, proteins, and small molecules.

This section provides background information related to the present disclosure which is not necessarily prior art.

## Summary

This section provides a general summary of the disclosure, and is not a comprehensive disclosure of its full scope or all of its features.

In various aspects, the present disclosure provides a sensing method for determining a first concentration of a target analyte using a single-molecule sensor having a solid-state nanopore. The sensing method includes measuring a current signal of the solid-state nanopore over a predetermined period of time and using the measured current signal to identify first and second sets of distinguishable electrical signatures. The first set of distinguishable electrical signatures may be used to determine a first capture rate of the target analyte. The first capture rate may be a function of the first concentration and a first analyte property factor that exemplifies one or more physical properties of the target analyte. The second set of distinguishable electrical signatures may be used to determine a second capture rate of a control analyte having a second concentration. The second concentration may be known and the second capture rate may be a function of the second concentration and a second analyte property factor that exemplifies one or more physical properties of the control analyte. The method may further include creating a first ratio of the first capture rate to the second capture rate and a second ratio of the second analyte property factor to the first analyte property factor and using the first and second ratios to determine the first concentration. The first concentration may be determined by multiplying the second concentrations by the first and second ratios.

In one aspect, the method may further include receiving the first and second analyte property factors and the second concentration.

In one aspect, the first and second analyte property factors may be equivalent and the second ratio may equal 1.

In one aspect, the one or more physical properties of the target analyte exemplified by the first analyte property factor may evidence one or more of a size, diffusion coefficient, electrophoretic mobility, and effective charge of the target analyte; and the one or more physical properties of the control analyte exemplified by the second analyte property factor may evidence one or more of a size, diffusion coefficient, electrophoretic mobility, and effective charge of the control analyte.

In one aspect, the first and second capture rates may be measured using a method selected from the group consisting of conductance blockage, passage time, electric charge deficit, number of distinct sublevels, event shape, event frequency spectrum, and combinations thereof.

In one aspect, at least one of the target analyte and the control analyte may include a bound proxy label.

In one aspect, the proxy label is cleaved from the at least one of the target analyte and the control analyte prior to detection.

In various other aspects, the present disclosure provides a precise sensing method for measuring an unknown concentration using a solid-state nanopore that simultaneously translocates a first analyte and a second analyte. The first analyte has an unknown concentration, and the second analyte has a known concentration. The sensing method may include receiving a current signal from the solid-state nanopore over a predetermined period of time and using the current signal to determine first and second capture rates. The first and second capture rates may each be a function of a pore property factor that represents one or more physical properties of the solid-state nanopore. The first capture rate may be further a function of a first analyte property factor that represents one or more physical properties of the first analyte and the unknown concentration. The second capture rate may be further a function of a second analyte property factor that represents one or more physical properties of the second analyte and the known concentration. The method may further include creating a ratio of the first capture rate to the second capture rate and determining the unknown concentration using the ratio such that the ratio cancels any effect of the physical properties of the solid-state nanopore on the concentration determinations.

In one aspect, the method may further include receiving the first and second analyte property factors and the second concentration.

In one aspect, the first and second analyte property factors may be equivalent and a ratio of the first analyte property factor to the second analyte property factor may be 1.

In one aspect, the one or more physical properties of the first analyte represented by the first analyte property factor may evidence one or more of a size, diffusion coefficient, electrophoretic mobility, and effective charge of the first analyte; and the one or more physical properties of the second analyte represented by the second analyte property factor may evidence one or more of a size, diffusion coefficient, electrophoretic mobility, and effective charge of the second analyte.

In one aspect, the one or more physical properties of the solid-state nanopore represented by the pore property factor may evidence one or more of the size, shape, surface charge density, and surface roughness of the solid-state nanopore.

In one aspect, the first and second capture rates may each be a further function of an operating factor that represents one or more operating conditions of the solid-state nanopore.

In one aspect, the ratio of the first capture rate to the second capture rate may cancel any effect of the operating conditions of the solid-state nanopore on the concentration determinations.

In one aspect, the first and second capture rates may be measured using a method selected from the group consisting of conductance blockage, passage time, electric charge deficit, number of distinct sublevels, event shape, event frequency spectrum, and combinations thereof.

In one aspect, at least one of the first analyte and the second analyte includes a bound proxy label.

In one aspect, the proxy label is cleaved from the at least one of the target analyte and the control analyte prior to detection.

In various other aspects, the present disclosure provides a precise sensing method for measuring an unknown concentration using a solid-state nanopore that simultaneously translocates a first analyte and a second analyte. The first analyte has an unknown concentration, and the second analyte has a known concentration. The method includes receiving a current signal from the solid-state nanopore over a predetermined time period and using the current signal to determine first and second population counts. The first population count is a function of a single-molecule count of the first analyte, and the second population count is a function of a single-molecule count of the second analyte. The method further includes creating a ratio of the first population count to the second population count and determining the unknown concentration using the ratio. The first concentration may be determined by multiplying the second concentration by the population count ratio.

Further areas of applicability will become apparent from the description provided herein. The description and specific examples in this summary are intended for purposes of illustration only and are not intended to limit the scope of the present disclosure.

## Brief Description of Embodiments

The drawings described herein are for illustrative purposes only of selected embodiments and not all possible implementations, and are not intended to limit the scope of the present disclosure.

FIG. 1 is a schematic illustration of an electric field around a conventional nanopore-based electrophoretic capture system;

FIG. 2A illustrates the number of translocations as a function of time for two nanopores having different diameters;

FIG. 2B illustrates the concentration-normalized capture rate as a function of DNA length for a nanopore sensor having an applied voltage of 300 mV and an initial pore diameter of 7 nm that is immersed in a 1.8 M LiCl solution having a pH of 8;

FIG. 2C illustrates the relative capture rates of two dsDNA samples having different lengths as a function of voltage in different nanopore systems having varying pore diameters;

FIGS. 3A-3C illustrate example methods for extracting a capture rate of one or more analytes from current signal of a nanopore sensor;

FIG. 4A illustrates the open pore current baseline as a function of time for a nanopore sensor that captures and translocates dsDNA having a length of 10 kbp, the sensor has an applied voltage of 300 mV and an initial pore diameter of 9 nm and is immersed in a 1.8 M LiCl solution having a pH of 8;

FIG. 4B illustrates the single molecule count as a function of time for the same system of FIG. 4A;

FIG. 5 illustrates the single molecule count and open pore current as a function of time for a nanopore sensor that translocates dsDNA having a length of 1.5 kbp, the sensor has an applied voltage of 200 mV and an initial pore diameter of 11 nm and is immersed in a 3.6 M LiCl solution having a pH of 8 ;

FIG. 6 illustrates the normalized capture rates as a function of voltage for three comparable nanopore sensors that capture and translocate dsDNA of variable lengths, each sensor having initial pore diameters of 7±1 nm and membrane thicknesses of 6±1 nm and immersed in a 1.8 M LiCl solution having a pH of 8;

FIG. 7A illustrates the normalized capture rates as a function of DNA length for 13 distinct nanopore sensors having initial pore diameters of 7±1 nm and membrane thicknesses of 6±1 nm that are each immersed in a 1.8 M LiCl solution having a pH of 8;

FIG. 7B is a histogram of the normalized capture rate distribution for all combined dsDNA lengths from the diffusive regime samples of FIG. 7A;

FIG. 8 is a schematic illustration of an electrophoretic capture system in accordance with various aspects of the present disclosure;

FIG. 9A illustrates the normalized capture rates as a function of time for two distinct analytes (250 bp DNA and 1.5 kbp DNA) in a nanopore sensor having an initial pore diameter of 11 nm and that is immersed in a 3.6 M LiCl solution having a pH of 8;

FIG. 9B illustrates the normalized capture rates of FIGS. 7A-B on opposing axes and a 95% prediction interval as predicted by a simple linear regression;

FIG. 10 is a flowchart illustrating an example method for determining an unknown concentration of a target analyte using a single-molecule sensor having a solid-state nanopore and a second analyte having a known concentration;

FIG. 11A illustrates a capture rate ratio as a function of a concentration ratio for two distinguishable DNA populations;

FIG. 11B illustrates a normalized capture rate ratio as a function of a DNA length ratio;

FIG. 11C is a histogram of the capture rate ratio of FIG. 11B;

FIG. 12A illustrates a population count ratio as a function of concentration ratio for the same distinguishable DNA populations illustrated in FIG. 11A;

FIG. 12B illustrates a normalized population count ratio as a function of DNA length ratio; and

FIG. 12C is a histogram of the population count ratio of FIG. 11B.

Corresponding reference numerals indicate corresponding parts throughout the several views of the drawings.

Example embodiments will now be described more fully with reference to the accompanying drawings.

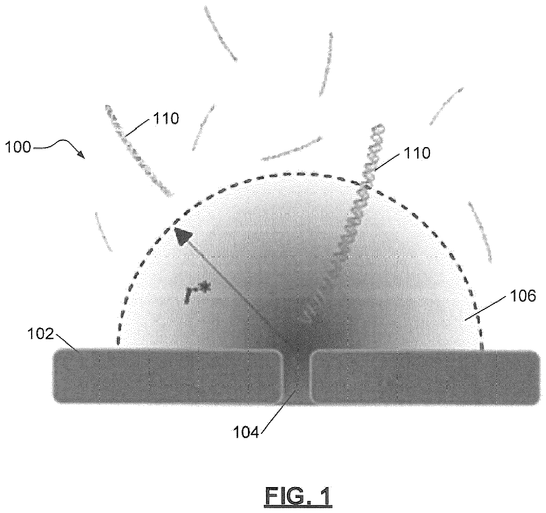

The concentrations of an analyte (e.g., biomolecule) in a liquid sample can be measured by the rate or frequency at which instances of the analyte translocate a solid-state nanopore. For example, FIG. 1 is a schematic illustration of a conventional nanopore-based electrophoretic capture system 100 for measuring the concentrations of an analyte or molecule 110. The capture system 100 comprises a solid-state membrane 102 having a nanopore 104 and is subjected to an applied voltage that induces an electric field distribution or electric field gradient 106. The electric field gradient 106 is oriented to promote entry of the analyte 110 into and through the nanopore 104.

The capture and translocation of the analyte 110 through the nanopore 104 under applied voltage is broadly categorized in three steps: (1) at a distance ($r^*$) from the pore, motion of the analyte 110 transitions from a pure diffusive motion state to an electrophoretically-dominated drift motion state in which the electric field distribution 106 directs or carries the analyte 110 towards the nanopore 104; (2) upon entering the proximity of the nanopore 104-a distance comparable to the size of the analyte 110-the analyte 110 finds a configuration that is conducive to the analyte 110 entering the nanopore 104; and (3) the analyte 110 translocates through the nanopore 104 or retracts and drifts away from the nanopore 104 and again becomes part of the bulk solution.

More specifically, applying a voltage difference across the nanopore 104 induces an electric field distribution or gradient 106 both within and around the nanopore 104, resulting in an electric field gradient having spherical symmetry and an approximate inverse square dependence on distance from the nanopore 104. The translocation or capture process depends on the nature of the electric field gradient surrounding the nanopore 104, as well as how the analyte 110 responds to the gradient. The response of the analyte 110 can be characterized by an electrophoretic mobility factor ($\mu$) that reflects a balance between electrophoretic forces driving the analyte 110 towards the nanopore 104 and viscous drag caused by counter-ions and charged molecules in solution with the analyte 110. In this fashion, the mobility of the analyte 110 can be affected by various extrinsic conditions, such as temperature, and salt concentrations. In various instances, such extrinsic parameters can be generally controlled and kept fixed.

The electric field gradient 106 can be approximated using a combination of Ohm's law ($E = J/\sigma$, where $J$ is the current density through the nanopore 104 and $\sigma$ is the solution conductivity) and hemispherical symmetry ($J = I/2\pi r^2$, where $I$ is the current through the nanopore 104 and $r$ is the radial distance away from the nanopore 104):

$$E(r) = \frac{I}{2\sigma\pi r^2} = \frac{G\Delta V}{2\sigma\pi r^2} \qquad \text{Eq. I.}$$

G is the nanopore conductance that codes all geometric information about the nanopore 104, including its diameter (*d*) and height or length (*h*), and $\Delta V$ is the applied voltage.

The dependence of the electric field gradient 106 on the geometric and surface properties of the nanopore 104 are represented by the nanopore conductance term (G). In high-sait environments (where surface effects are minimized) and at radial distances (r) from the nanopore 104 defined by $r > d/2$, the conductance (G) of a cylindrical shaped nanopore 104 can be modeled by a bulk pore conductance in series with conributions from two hemispherical access-region conductance (*e.g.*, $G^{-1} = 2G_{acc}^{-1} + G_{cyl}^{-1}$). In such instances, the electric field gradient 106 can, therefore, be approximated as:

$$E(r) = \left(\frac{d^2\Delta V}{8h+2\pi d}\right)\frac{1}{r^2} \qquad \text{Eq. II.}$$

For high-aspect ratio (*i.e.*, $h > d$) nanopores, the electric field gradient at radical distances ($r > d/2$) can be approximated as $E(r) \approx d^2\Delta V/8hr^2$. However, such an approximation is not accurate for thin membranes (*e.g.*, $h \lesssim 10$ nm) having aspect ratios ($d/h$) close to 1.

The capture radius ($r^*$) can be approximated as the distance at which the radial diffusive velocity (e.g., $v_d(r) = D/r$) of the analyte 110 (having diffusion constant ($D$)) is balanced by the electrophoretic drift (e.g., $v_E(r) = \mu E$) induced by the electric field gradient 106 on the analyte 110 (having electrophoretic mobility ($\mu$)):

$$r^* = \frac{\mu G\Delta V}{\sigma 2\pi D} \qquad \text{Eq. III}$$

When the analyte 110 comprises linear DNA, the diffusion constant (D) can be approximated by $D \propto L^v$, where L is the DNA length and $v$ is the Flory exponent, and the electrophoretic mobility ($\mu$) can be approximated by $\mu \propto L^0$.

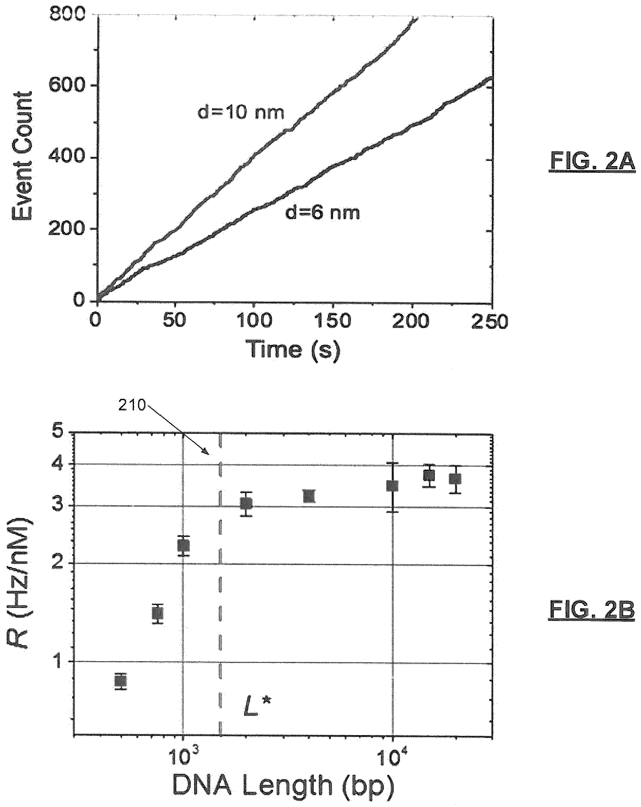

The capture radius ($r^*$) will be different for target analytes 110 having different lengths. For example, target analytes having larger, longer, or more highly charged molecules will have a larger capture radii compared to target analytes having smaller, shorter, or more charge-neutral molecules. Similarly, nanopores having a larger diameter (*d*) and, therefore, stronger and more spatially extended electric field gradient 106 outside the pore have larger capture radii ($r^*$) and capture rates (J). For example, FIG. 2A illustrates the number of capture events as a function of time for two nanopores having different diameters. As illustrated, the capture rate for the larger pore ($d$ = 10 nm) is greater than the capture rate for the smaller pore ($d$ = 6 nm) at the same concentration (*e.g.*, 0.2 nM) of DNA having a length of 10 kbp under an applied voltage of 300 mV.

Translocation of the analyte 110 through the nanopore 104 requires the analyte 110 to perturb and confine itself from its free solution equilibrium. Requirements for overcoming the entropically-natured free-energy barrier of the free solution equilibrium gives rise to two regimes: (1) the diffusion-limited capture regime and (2) the barrier-limited capture regimes.

The diffusion-limited regime refers to instances where the entropic barrier is insignificant compared to the electrical potential energy pulling the analyte 110-i.e., F << qE. In such instances, successful translocation occurs once the analyte 110 enters the capture radius ($r^*$). The rate of translocation ($J_{diff}$) in the diffusion-limited regime, is equivalent to the rate at which the analyte 110 diffuse into the hemisphere defining the capture radius ($r^*$) and can be determined by solving the diffusion equation in the presence of an absorbing hemisphere:

$$J_{diff} = 2\pi c D r^* \qquad \text{Eq. IV}$$

where c is the concentration of the analyte 110. In the diffusion-limited regime, the diffusion-limited capture rate ($J_{diff}$) is directly proportional to the applied voltage (*i.e.*, linear voltage dependency).

The barrier-limited regime refers to instances where the entropic barrier is sufficiently large compared to the electrical potential energy pulling the molecule causing target molecules 110 to drift away from the nanopore 104 before successfully translocating the nanopore 104 and may necessitate several translocation attempts prior to successfully translocating the nanopore 104. The barrier-limited capture rate ($J_{bar}$) can be defined using a Kramer or Arrhenius process:

$$J_{bar} = \omega \exp\left[-\frac{F_b - F_E}{k_B T}\right] \qquad \text{Eq. V}$$

where $F_b$ is the height of the energy barrier, $F_E$ is the electrostatic free energy of the molecule in the electric field (E), and $\omega$ is the barrier-crossing attempt rate. In the barrier-limited regime, the barrier-limited capture rate ($J_{bar}$) is exponentially proportional to the applied voltage (*i.e.*, exponential voltage dependency).

In the instance of an analyte 110 comprising linear dsDNA polymer, the diffusion-limited capture rate ($J_{diff}$) is independent on the length of the dsDNA polymer and the barrier-limited capture rate ($J_{bar}$) is dependent on the length of the dsDNA polymer. For example, as illustrated in FIG. 2B, the barrier-limited regime is observed for small molecules ($\lesssim$ 2 kbp) and the length independence of the diffusion-limited regime is observed for longer molecules ($\gtrsim$ 2 kbp). The DNA length at which the two regimes cross is represented by vertical line 210. The crossover length ($L^*$) is dependent on various experimental parameters, such as the strength of the attracting field and pore geometry.

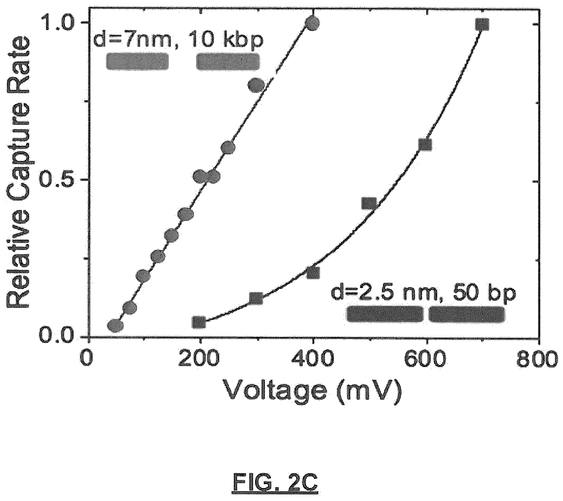

FIG. 2C highlights the voltage dependencies of the barrier-limited and diffusion-limited regimes by comparing the relative capture rates of first and second samples in different nanopore systems. The first sample comprises dsDNA having a length of 10 kbp in a 1.8 M LiCl solution. The second sample comprises dsDNA having a length of 50 bp in a 3.6 M LiCl solution. The first sample (circles) is measured by a nanopore sensor having a pore diameter of 7 nm, and the second sample (squares) is measured by a nanopore sensor having a pore diameter of 2.5 nm. In each instance, the barrier-limited regime is observed at lower voltages and the diffusion-iimited regime is observed at higher voltage (not shown). Similar to crossover length ($L^*$), the crossover voltage ($V^*$) is dependent on various experimental parameters, such as pore geometry and sample length. For example, longer dsDNA results in smaller $V^*$ values.

Generally, the electrophoretic-based capture rate of dsDNA linear polymer in both the barrier-limited and diffusion-limited regimes can be defined using the following equation:

$$J = RC = \begin{cases} \frac{G\mu\Delta V c}{\sigma}; & L > L^*, V > V^* \\ \beta_1 e^{\beta_2 \Delta V} L^{\alpha} c; & L < L^*, V < V^* \end{cases} \qquad \text{Eq. VI}$$

The pore conductance (G) represents the pore geometry dependence in the diffusion-limited regime and $\beta_1$, $\beta_2$, and $\alpha$ are scaling factors related to various experimental conditions and nanopore properties in the barrier-limited regime. In this fashion, the diffusion-limited and barrier-limited regimes are special cases of the Smoluchowski differential equation solution. In various instances, a purely electrophoretic capture model suffices in outlining dependencies of capture rate on intrinsic (e.g., pore geometry, electric field) and extrinsic (e.g., temperature, salt concentration) experimental conditions. According to this electrophoretic capture framework, translocation frequency and open-pore conductance are two manifestations of the same underlying mechanism-*e.g.*, the transport of charged molecules through an electric field having a profile depending on the system geometry.

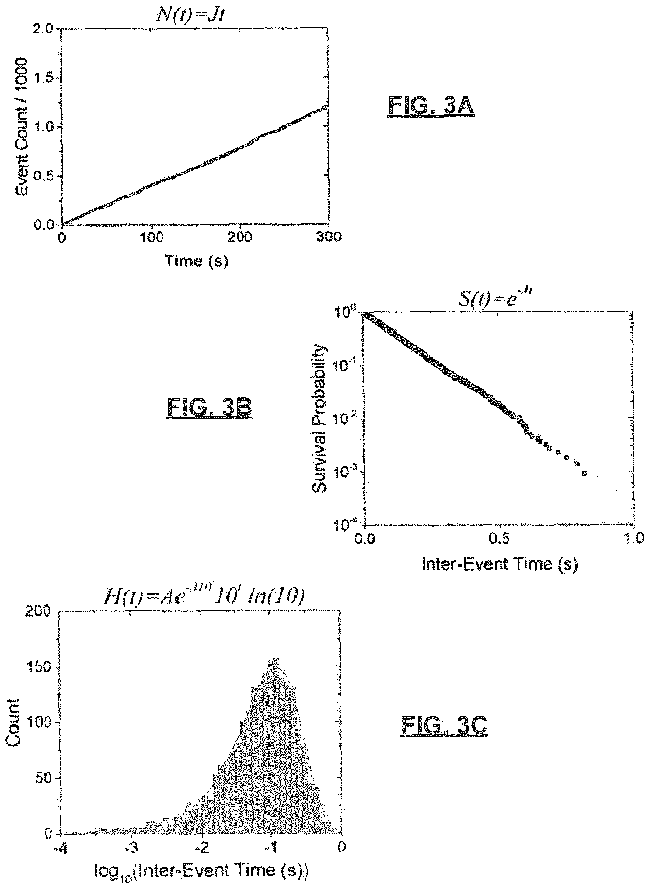

With renewed reference to FIG. 1, the capture rate (*J*) of the target analyte 110 can be extracted from a measured signal current of the nanopore 104. For example, in certain instances, as illustrated in FIG. 3A, the capture rate (*J*) can be obtained from the slope of the cumulative single molecule translocation event counts as a function of time. In other instances, as illustrated in FIG. 3B, the capture rate (*J*) can be obtained from the statistical analysis of inter-event times as calculated by the survival probably. In still further instances, as illustrated in FIG. 3C, the capture rate (*J*) can be obtained by fitting a distribution of the log of inter-event times. In various aspects, the capture rate (*J*) can be measured using a method selected from the group consisting of conductance blockage, passage time, electric charge deficit, number of distinct sublevels, event shape, event frequency spectrum, and combinations thereof.

In various aspects, the molecular concentration of the analyte 110 in solution can be determined by measuring the rate or frequency (*J*) at which the target analyte translocates the nanopore 104. More specifically, in dilute solutions where inter-molecular interactions are considered insignificant, the extracted capture rate (*J*) is linearly proportional to the concentration (c) of the analyte 110:

$$J = R \cdot c \qquad \text{Eq. VII}$$

where R is a normalized capture rate. The normalized capture rate (R) is dependent on the geometry of the nanopore capture system 100 and the electrophoretic transport properties of the analyte 110 and can be determined using a calibration curve. Such a calibration curve may be created by measuring the capture rate (*J*) for a variety of concentrations ($c_i$, where $i > 1$). The calibration curve is in principle valid for any identical nanopore under identical conditions.

The precision of the nanopore concentration determination is thus dependent on both the ability to extract the normalized capture rate (R) from the calibration curve and the ability to consistently fabricate nanopore capture systems with identical capture properties and systematically operate these capture systems under identical conditions (*i.e.*, controlling extrinsic parameters), which is not feasible under real-world conditions using state-of-the art nanofabrication tools. Further, there are additional dependencies in real-world nanopore measurements that may cause variations in the extracted capture rate. Such variations may occur between nominally identical pores (*i.e.*, inter-pore variation(s)), as well as within a single nanopore over time (*i.e.*, intra-pore variation(s)).

There are several potential sources for these variations. Some of the potential sources for variation can be controlled with varying degrees of precision by the user-such as, salt concentration (±2%), applied voltage (±1mV), nominal membrane thickness (±1 nm), and initial pore size (±1 nm). However, in practice even nanopores having similar dimensions and operated under similar environmental conditions can display significantly different capture characteristics when operated upon similar or identical experimental parameters. That is, as identified nominally identical nanopores may not have the same, or even similar, normalized capture rate (R) as nanoscopic differences can exist between pores that are not readily discernable from measurement techniques.

Further still, many of the potential sources for variation cannot be easily controlled, including for example, effective pore length, precise pore geometry, and pore size stability. Nanopores with identical conductance and electrical characteristics can have widely varying geometries that may affect the capture rate between nanopores that appear otherwise electrically identical. In certain instances, capture rates may also be dependent upon the local details in and around the solid-state nanopore. For example, electroosmotic flow induced by membrane surface charges has been shown to impact the capture and translocation properties of various charged molecules and, in some instances, to completely prevent translocation. Likewise, electrostatic interactions may contribute to forces felt by target analytes approaching the charged nanopore surface. For example, polymer-polymer electrostatic repulsion caused by molecules sticking to the membrane near the nanopore may cause changes in the pore conductance and capture rate.

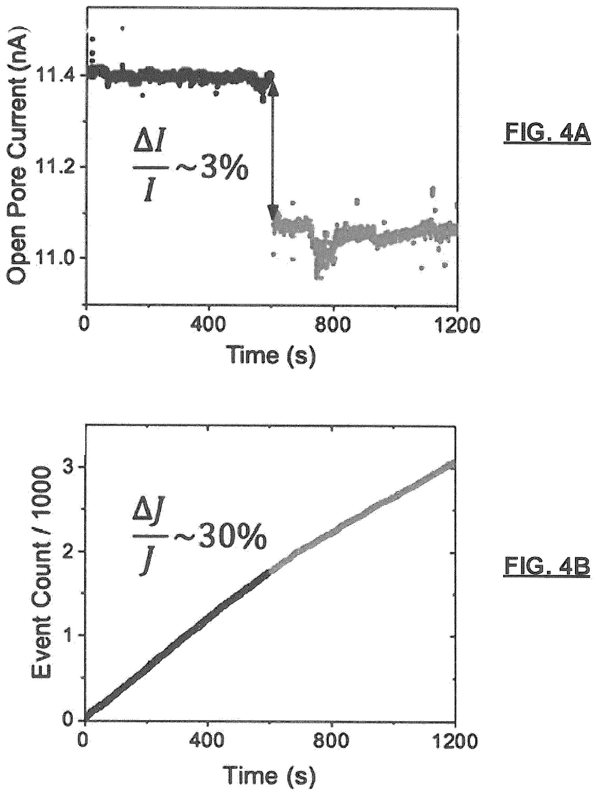

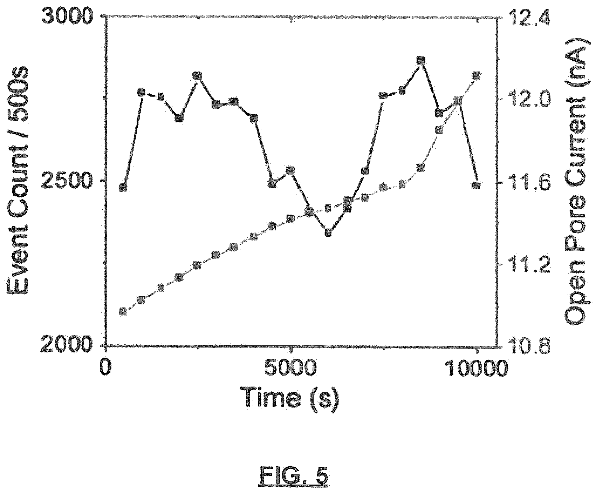

FIGS. 4A-4B and 5 highlight potential intra-pore variabilities. More specifically, FIG. 4A illustrates the open pore current (nA) for a nanopore sensor having an initial pore diameter of 6±1 nm and immersed in a 1.8 M LiCl solution having a pH of 8. A voltage of about 300 mV was applied over the course of about 20 minutes and the nanopore sensor captured and translocated dsDNA having a length of 10 kbp. As illustrated, after about 600 seconds of steady baseline, an about 3% decrease in open pore current was observed. The 3% decrease represents an approximate 350 pA change, which is smaller than the blockage level (about 850 pA) expected as a result of dsDNA caught inside the nanopore under the same experimental conditions. FIG. 4B illustrates the single molecule count as a function of time (s) for the same dsDNA and shows a coincident about 30% decrease in the capture rate.

FIG. 5 illustrates the single molecule count and the open pore current (nA) as a function of time (s) for a nanopore sensor that captured and translocated dsDNA having a length of 1.5 kbp. The nanopore sensor has an initial pore diameter of 11 nm and was immersed in 3.6 M LiCl solution having a pH of 8. A voltage of about 200 mV was applied over the course of about 3 hours. As illustrated, a capture rate variation of about 20% was observed throughout the experiment. The open pore current is superimposed to illustrate that the capture rate variation is in this case uncorrelated to the open-pore current variation.

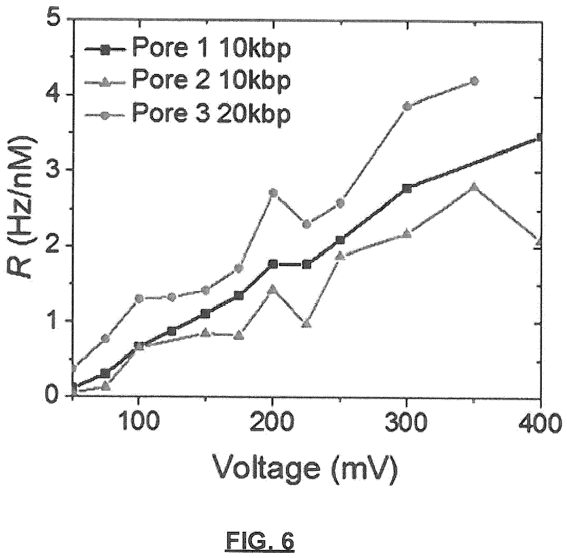

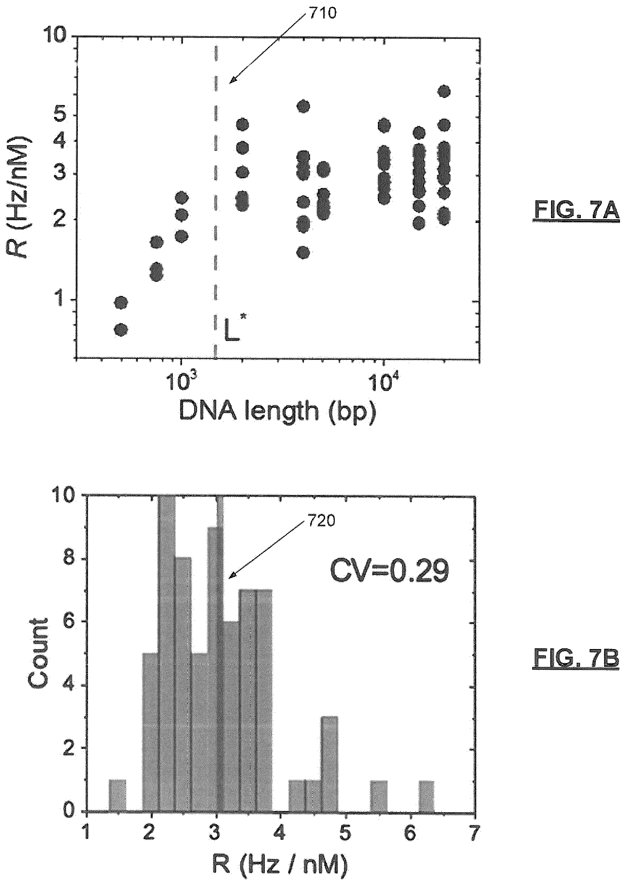

FIGS. 6 and 7A-7B highlight inter-pore variability. More specifically, FIG. 6 illustrates the concentration-normalized capture rates (Hz/nM) as a function of voltage (mV) of three comparable nanopore sensors having initial pore diameters of 7±1 nm and membrane thicknesses of 6±1 nm and immersed in 1.8 M LiCl solution having a pH of 8. For two of the three nanopores DNA having a length of 10 kbp is captured and translocated. The results for the first nanopore (*i.e.*, Pore 1) are illustrated using rectangles, and the results for the second nanopore (*i.e.*, Pore 2) are illustrated using triangles. For the third nanopore, DNA having a length of 20 kbp is captured and translocated. The results for the third nanopore (*i.e.*, Pore 3) are illustrated using circles. Because the dimensions are similar and the DNA lengths are the same and the diffusion-limited regime assumes a length independent capture rate, the normalized capture rates for the first and second nanopores should be the same. However, as illustrated, the normalized capture rates for Pore 1 and Pore 2 are different. For example, at 300 mV, Pore 1 has a capture rate of about 2.9 Hz/nM and Pore 2 has a capture rate of about 2.2 Hz/nM. Also, because the diffusion-limited regime assumes a length independent capture rate, and since the dimensions of Pore 1, Pore 2, and Pore 3 are the same, the normalized capture rates for the first, second, and third nanopores should be the same. However, as illustrated, the normalized capture rates for Pore 1, Pore 2, and Pore 3 are different. For example, at 300 mV, Pore 3 has a capture rate of about 3.9 Hz/nM, while Pore 1 has a capture rate of 2.9 Hz/nM and Pore 2 has a capture rate of about 2.2 Hz/nM.

FIG. 7A illustrates the normalized capture rates (Hz/nM) as a function of DNA length (bp) of 13 distinct nanopore sensors, each fabricated with the same initial pore diameter ($d$ = 7±1 nm) and effective membrane thickness ($L$ = 6±1nm) and immersed in a 1.8 M LiCl solution having a pH of 8. A voltage of about 300 mV is applied to the nanopores. The vertical line 710 roughly distinguishes the barrier-limited and the diffusion-limited regimes. The barrier-limited regime appears to the left of the vertical line 710 and the diffusion-limited regime appears to the right of the vertical line 710. FIG. 7B is a histogram of the normalized capture rate distribution for all combined dsDNA lengths from the diffusive regime, where the capture rates have no dependence on polymer length. A significant spread is observed that corresponds to a 0.29 coefficient of variation. Such a variation is not expected to be caused by simple geometrical variations because the respective capture rates and open pore conductance are only weakly correlated (r = 0.33). The vertical line 720 outlines a mean normalized capture rate of about 3.08 Hz/nM with a coefficient of variation of about 0.29.

To overcome these inter-pore and intra-pore sources of error, in various aspects, the present disclosure provides a controlled counting method that relies on simultaneously sensing a second molecular population having a precisely known concentration that acts as an internal calibrator. The second molecular population has an easily distinguishable electrical signature from that of the first target molecule or analyte. As further detailed below, the respective capture rates of both the first and second analytes are highly correlated in time, as well as from pore to pore, despite the uncontrolled sources of variability highlighted above.

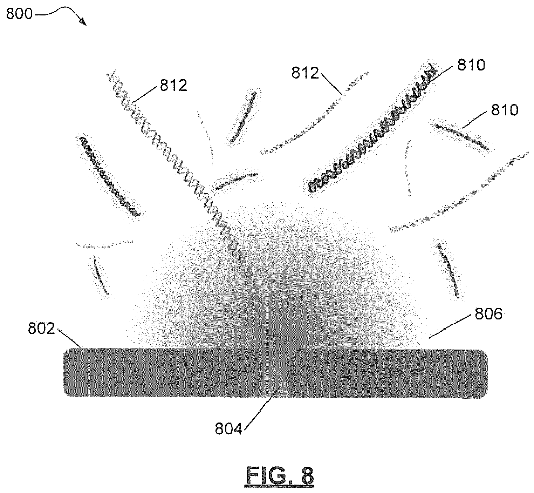

The high correlation enables improved precision in concentration measurements through calculating the ratio of capture rates of both the first and second molecular populations. The capture rate ratio reduces measurement uncertainty and removes the need to employ pores with identical and time-invariant characteristics, thereby eliminating the dependency on uncontrolled nanopore properties. For example, FIG. 8 illustrates an electrophoretic capture system 800 for simultaneously measuring the concentrations of a target or first analyte 810 and a control or second analyte 812. The nanopore sensor 800 comprises a solid-state membrane 802 having a nanopore 804. The capture radius 806 is determined in a manner similar to that illustrated in FIG. 1.

In various aspects, the normalized capture rate (R) can be depicted as a function of the one or more physical properties of the nanopore (P) and one or more physical properties of the target analyte (Q):

$$J = R * c = P * Q * c \qquad \text{Eq. VIII.}$$

The one or more physical properties (Q) of the target analyte 810 refers to, for example only, the dimensions and electrostatic properties of the target analyte 810, as well as the electrophoretic mobility factor ($\mu$) and diffusion coefficient. The one or more physical properties (Q) of the target analyte 810 are independent of the physical properties of the nanopore 804 and are time-constant in instances of fixed or time-constant experimental conditions. The one or more physical properties (P) of the nanopore 804 refers to, for example only, the dimensions and electrostatic properties of the nanopore 804, including its size and shape, as well as the surface charge density and surface roughness.
Each nanopore 804 has a unique P-value that may be time variable. However, the first and second analytes 810, 812 can be selected such that the same P-valve will apply for both analytes. For example, the first and second analytes 810, 812 may be two different lengths of linear dsDNA. In such instances, the physical and/or chemical properties of the particular nanopore 804 will similarly affect both the target and analytes of known concentration 810, 812. That is, if a molecule has a higher capture rate than expected due to uncontrolled errors, any other molecular species (e.g., analytes 810, 812) present in the solution will also have a higher capture rate.

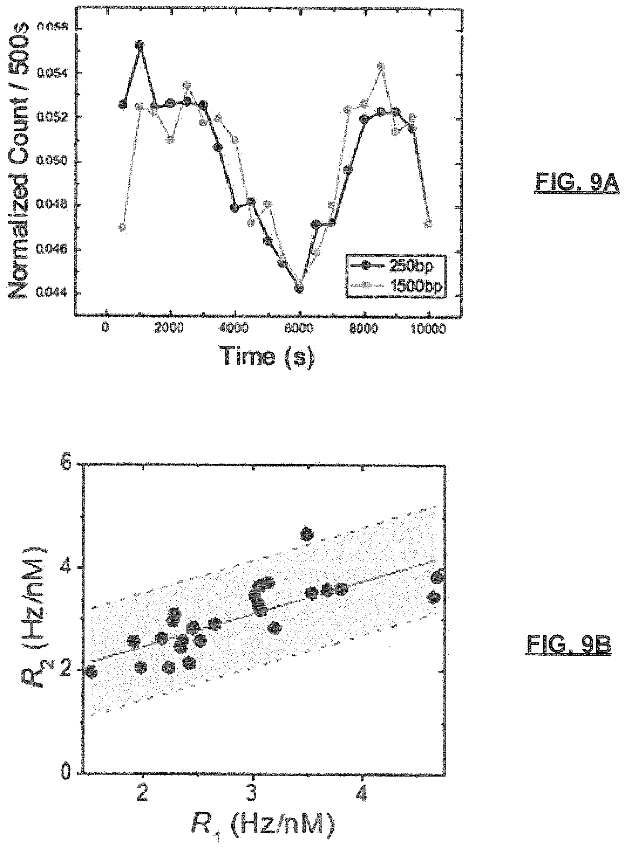

For example, FIG. 9A illustrates the time evolution over the course of an about 3 hour time period of single-molecule translocation counts of a mixture of a first DNA analyte having a length of 250 bp and a second DNA analyte having a length of 1.5 kbp in a 3.6 M LiCl solution by a nanopore sensor having a pore diameter of about 11 nm. As illustrated, the two DNA types have well distinguished event characteristics. However, the DNA types show highly correlated intra-pore capture variations, which demonstrates the fact that intra-pore capture rate variations fluctuations originate from time-varying nanopore properties (P) and not time-insensitive analyte properties (Q). Further, FIG. 9B, using the diffusive-regime capture rate date from the 13 nanopore sensors analyzed in FIGS. 7A-B, where every experiment involved the mixture of two distinguishable dsDNA lengths, illustrates the normalized capture rates on the opposing axes. A strong correlation (r = 0.77) between the normalized capture rates is observed.

In this fashion, the capture rate ($J_2$) of a second analyte 812 having a known concentration ($c_2$) can be correlated

with the capture rate ($J_1$) of a first analyte 810 having an unknown concentration. Such correlations can be used to improve the precision of nanopore concentration measurements. If a second population (*i.e.*, a second analyte) 812 having a known concentration ($c_2$) is present at the same time as the target population (*i.e.*, first or target analyte) 810, the second population 812 can be used as an internal calibrator to normalize the effect of uncontrolled pore properties (P), which similarly effect both the first and second analytes. For example, in various aspects, the first capture rate ($J_1$) is depicted as:

$$J_1 = PQ_1c_1 \qquad \text{Eq. IX,}$$

while the second capture rate ($J_2$) is depicted as:

$$J_2 = PQ_2c_2 \qquad \text{Eq. X.}$$

Since the capture rates of both the first and second analytes 810, 812 depend on the same pore-specific function (P) at any time during an experiment, the unknown concentration can be determined using the following ratio:

$$c_1 = \frac{J_1Q_2c_2}{J_2Q_1} \qquad \text{Eq. XI.}$$

In various instances, the target or first analyte may comprise linear dsDNA polymer. In such instances, a diffusion-limited regime may be observed when conditions are as discussed above. That is, the energy barrier of the free solution may be insignificant compared to the electrical potential energy of nanopore and related capture radius that pulls the target molecule and $Q_1$ and $Q_2$ may be equivalent, such that $Q_1/Q_2 \sim 1$. The unknown concentration can therefore be determined simply by multiplying the ratio of first and second capture rates by the known concentration:

$$c_1 = \frac{J_1}{J_2}c_2 \qquad \text{Eq. XII.}$$

In certain aspects, the first and second capture rates ($J_1$ and $J_2$) may be further a function of an operating factor that represents the conditions in which the capture rates are measured.

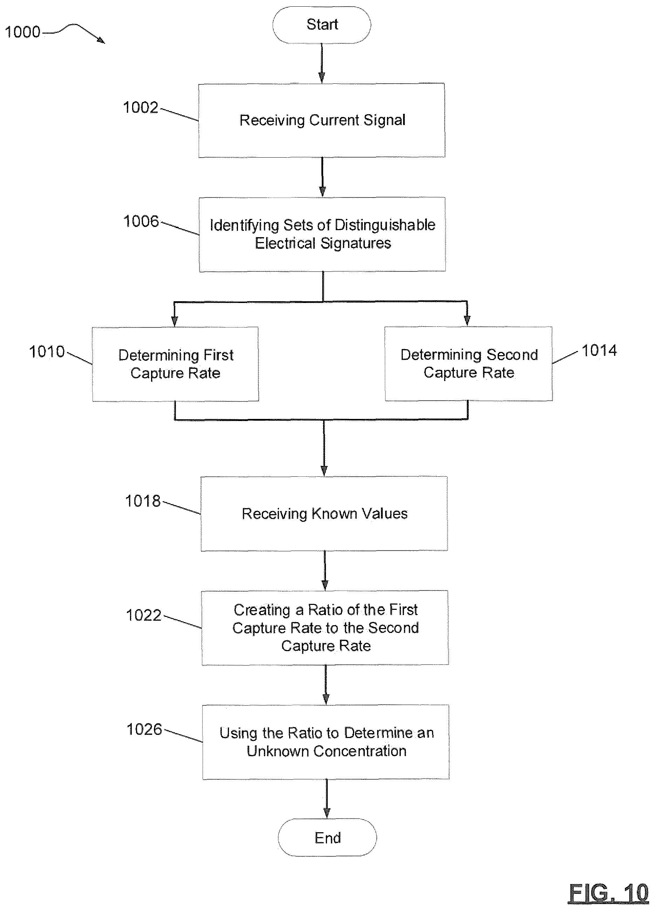

FIG. 10 is a flowchart illustrating an example method 1000 for determining the unknown concentration ($c_1$) of a target analyte using a single-molecule sensor having a solid-state nanopore and a second analyte having a known concentration. The method includes at 1002 receiving current signal from the solid-state nanopore over a predetermined period of time. At 1006, the received current signal is used to identify first and second sets of distinguishable electrical signatures.

At 1010, the first set of distinguishable electrical signatures is used to determine a first capture rate ($J_1$) of the target analyte. At 1014, the second set of distinguishable electrical signatures is used to determine a second capture rate ($J_2$) of the control analyte. In various aspects, the determination of the first capture rate ($J_1$) at 1010 and the determination of the second capture rate ($J_2$) at 1014 may occur in parallel. In certain aspects, not shown, the determination of the first capture rate ($J_1$) at 1010 and the determination of the second capture rate ($J_2$) at 1014 may occur serially.

In various instances, the method includes at 1018 receiving the first and second analyte property factors ($Q_1$ and $Q_2$) and/or the known concentration value ($c_2$). At 1022, a ratio of the first capture rate to the second capture rate is calculated, cancelling out the any effect of the physical properties of the solid-state nanopore and in some instances the first and second analyte property factors. See Equations XI and XII. The ratio of the first capture rate to the second capture rate is then used at 1026 to determine the unknown concentration ($c_1$). In certain aspects, not shown, the method may be cyclic returning to 1002 following the determination of the unknown concentration ($c_1$).

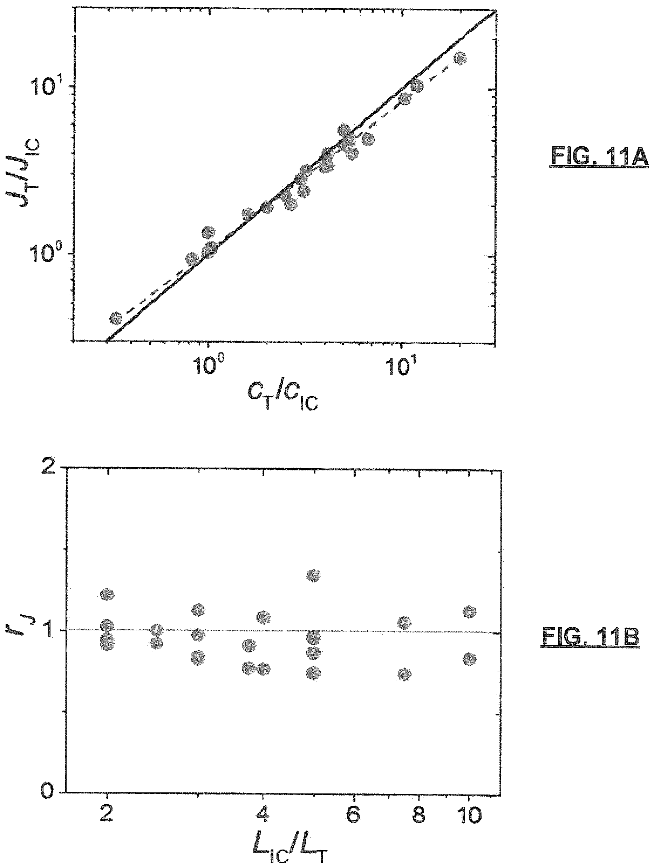

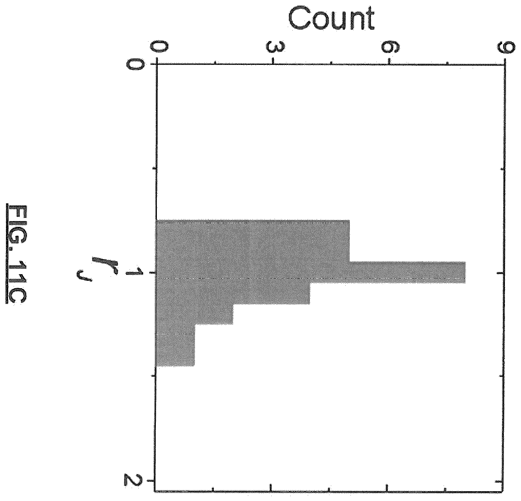

FIGS. 11A-11C provide an example validation of this method. More particularly, FIG. 11A illustrates a capture rate ratio as a function of a concentration ratio for two distinguishable DNA populations. The solid line in FIG. 11A shows the expected relation y=x and the power law exponent is 0.88 ± 0.05. FIG. 11B illustrates a ratio of the normalized capture rates ($r_J = \frac{R_1}{R_2} = \frac{J_1c_2}{J_2c_1}$) as a function of DNA length ratio ($L_1/L_2$). As illustrated, the ratio of normalized capture rates to DNA length ratios demonstrates no clear length dependence. The ratio values are all near 1, as required. FIG. 11C illustrates the distribution of $r_J$. As illustrated, the coefficient of variation is 0.16, which is a significant reduction from the previously observed 0.29. As such, using an internal calibrator of known concentration removes the influence of the specific nanopore sensor in use, allowing for more precise measurements of the unknown target concentration independent of uncontrolled sources of systematic error. In particular, in many aspects, using the internal calibrator may eliminate the need to use separate calibration curves and the need for precise pore geometry or geometric stability.

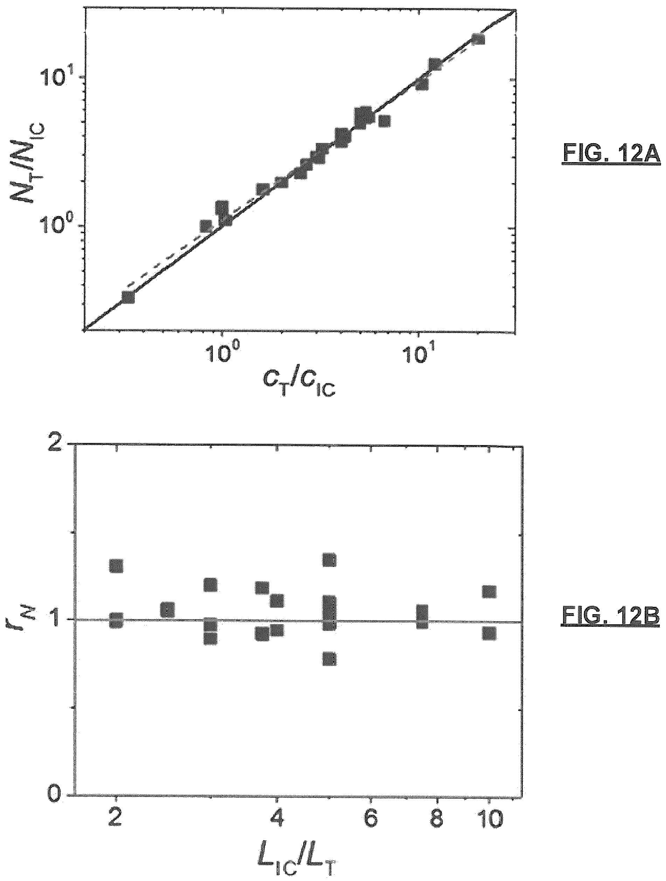

In various aspects, significant intra-pore variations are present in an experiment and the capture rate may become an ill-defined quantity (*see, e.g.,* FIG. 4A-4B and 5). In such instances, the calculation of capture rates by fitting inter-event times mistakenly assumes a constant normalized capture rate (R), resulting in the extraction of ill-defined capture rate values (J). Consequently, when the normalized capture rate (R) is not constant and the assumption does not hold, population counts ratio ($N_1/N_2$) may be preferable to capture rate ratios ($J_1/J_2$) because single-molecule counts intrinsically consider time variations of capture. For example, FIGS. 11A-11C are replotted in FIGS. 12A-12C using a population count ratio ($N_1/N_2$) in placed of the capture rate ratio ($J_1/J_2$).

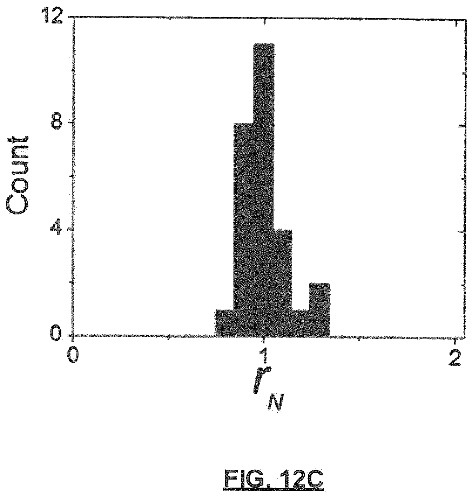

FIG. 12A illustrates a population count ratio as a function of a concentration ratio for the two distinguishable DNA populations of FIG. 11A. The solid line in FIG. 12A shows the expected relation y =x and the power law exponent is 0.94 $\pm$ 0.05, which is closer to the expected value of 1. FIG. 12B illustrates a ratio of the normalized population counts $(r_N = \frac{N_1 c_2}{N_2 c_1})$ as a function of DNA length ratio ($L_1/L_2$). FIG. 12B illustrates no correlation between the two ratios. FIG. 12C illustrates the distribution of $r_N$. As illustrated, the coefficient of variation is 0.12. As such, employing a ratio of population counts to determine the concentration of an unknown molecular target as opposed to the ratio of capture rate can further improve the precision of the measurements, particularly when intra-pore variations are present.

The most probable source of the increased coefficient of variation when using capture rate ratios instead of population count ratio may be the fact that the capture rates may vary in time, which adds additional uncertainty when a constant capture rate is assumed. Further, in various aspects, the coefficient of variation may be further reduced when using only a single predetermined and premeasured internal calibrator instead of a range of calibrators. Both $r_J$ and $r_N$ depend on four measured parameters, whereas the target concentration ($c_1$) calculated using Equation XI depends on only three measured parameters for the case of linear dsDNA. As such, the precision of concentration measurements made using the method summarized by Equation XI is further improved.

Aside from the internal control concentration uncertainty, the fundamental limit of precision for the measurement of capture rate ratios is ultimately Poisson noise, decreasing with the inverse square root of the number of events used to calculate the ratio. Note also that the polymer length transition between the barrier-limited and diffusion-limited regimes is probably not be as sharp as described by theory. Working with DNA lengths near that boundary will result in increased uncertainty, due to the underlying assumption that $Q_1 = Q_2$ for dsDNA, an assumption which will improve in the limit of $L/L^* >> 1$.

While nanopore sensors (*e.g.*, electrophoretic capture system 800) in various instances have been shown to be able to interrogate some proteins directly, the protocols are complex and the specificity of the nanopore signal may not be suitable for direct detection of complex biological samples. As such, in various aspects it may be preferable to use a proxy label-such as, DNA-as the actual unit that is detected, using an immunoassay style conversion of protein to proxy label employing specific antibodies. Such a conversion may be achieved using an ELISA style sandwich assay.

In such instances, a primary capture antibody for the target protein may be bound to a surface or a paramagnetic bead that traps the target molecules. The target molecules may then be further tagged with a proxy label such as linear dsDNA that is end-terminated with a secondary detection antibody for the target protein, forming a sandwich. The sample may then be washed while holding the target molecules and proxy labels in place, removing any unbound sample or background molecule. Finally, the proxy label can be released off the detector antibody using a restriction enzyme, photocleavable linkage site, or a strand mediated displacement approach. The proxy label concentration is measurable and directly corresponds with the target molecule concentration.

In certain aspects, such a proxy label concentration may suffer from sources of uncertainty. For example, the number of antibodies bound per magnetic bead or per unit area may difficult to control and even more difficult to measure. Further, the binding efficiency of the sandwich may be highly sensitive to local conditions, such as salt concentration. Wash steps may also remove some subset of targets along with the background, and the cleaving efficiency of the proxy label release is not perfect and is difficult to measure. All of these error sources may contribute to large uncertainties in the extracted concentration values.

In various aspect, as described above, these sources of error may be mitigated using an internal control. For example, two sets of target proteins, associated antibodies, and proxy labels may be used. The control molecule is not natively present in the sample and is introduced at a known concentration. The target or interest and the control molecule may each have similar properties (e.g., size, molecular weight, dissociation constant ($K_d$), etc.). As such, the binding to the beads of the two distinct antibodies is a competitive process. For example, because the $K_d$ values for the target protein and internal calibrator are similar, each will be identically sensitive to local conditions and will bind to the deposited antibodies in a ratio dependent only on the relative concentrations and $K_d$ values for each reaction. Further, wash steps will remove both the target and the control in equal proportion, and the cleaving efficiency of the proxy label will be the same for both the target and the control or calibrator. The capture rates and/or population count of both the calibrator and target surrogate labels may be determined and respective ratios may be used as described above to correct for error that may otherwise interfere with the precision of the measurement. As such, a method for improving measurement precision without needing to explicitly account for difficult to measure sources of error during the assay itself is provided.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A sensing method for determining a first concentration of a target analyte using a single-molecule sensor having a solid-state nanopore, the sensing method comprising:

   - measuring a current signal of the solid-state nanopore over a predetermined period of time and using the measured current signal to identify first and second sets of distinguishable electrical signatures;
   - a using the first set of distinguishable electrical signatures, determining a first capture rate of the target analyte, wherein the first capture rate is a function of the first concentration and a first analyte property factor that exemplifies one or more physical properties of the target analyte;
   - using the second set of distinguishable electrical signatures, determining a second capture rate of a control analyte having a second concentration, wherein the second concentration is known and the second capture rate is a function of the second concentration and a second analyte property factor that exemplifies one or more physical properties of the control analyte; and
   - creating a first ratio of the first capture rate to the second capture rate and a second ratio of the second analyte property factor to the first analyte property factor and using the first and second ratios to determine the first concentration, wherein the first concentration is determined by multiplying the second concentration by the first and second ratios.

2. The sensing method of claim 1, wherein the method further includes receiving the first analyte and second analyte property factors and the second concentration.

3. The sensing method of claim 1 or 2, wherein the first and second analyte property factors are equivalent and the second ratio equals 1.

4. The sensing method of at least one of the preceding claims, wherein

- the one or more physical properties of the target analyte exemplified by the first analyte property factor evidences one or more of a size, diffusion coefficient, electrophoretic mobility, and effective charge of the target analyte; and
- the one or more physical properties of the control analyte exemplified by the second analyte property factor evidences one or more of a size, diffusion coefficient, electrophoretic mobility, and effective charge of the control analyte.

5. The sensing method of at least one of the preceding claims, wherein the first and second capture rates are measured using a method selected from the group consisting of conductance blockage, passage time, electric charge deficit, number of distinct sublevels, event shape, event frequency spectrum, and combinations thereof.

6. The sensing method of at least one of the preceding claims, wherein at least one of the target analyte and the control analyte includes a bound proxy label.

7. The sensing method of claim 6, wherein the proxy label is cleaved from the at least one of the target analyte and the control analyte prior to detection.

8. A precise sensing method for measuring an unknown concentration using a solid-state nanopore that simultaneously translocates a first analyte and a second analyte, wherein the first analyte has an unknown concentration and the second analyte has a known concentration, the sensing method comprising:

- receiving a current signal from the solid-state nanopore over a predetermined period of time and using the current signal to determine first and second capture rates, wherein

  - the first and second capture rates are each functions of a pore property factor that represents one or more physical properties of the solid-state nanopore, and
  - the first capture rate is further a function of a first analyte property factor that represents one or more physical properties of the first analyte and the unknown concentration and the second capture rate is further a function of a second analyte property factor that represents one or more physical properties of the second analyte and the known concentration; and

- creating a ratio of the first capture rate to the second capture rate and determining the unknown concentration using the ratio such that the ratio cancels any effect of the physical properties of the solid-state nanopore on the concentration determinations.

9. The precise sensing method of claim 8, wherein the method further includes receiving the first analyte and second analyte property factors and the second concentration.

10. The precise sensing method of claim 8 or 9, wherein the first analyte and second analyte property factors are equivalent and a ratio of the first analyte property factor to the second analyte property factor is 1.

11. The precise sensing method of at least one of claims 8 to 10, wherein

- the one or more physical properties of the first analyte represented by the first analyte property factor evidences one or more of a size, diffusion coefficient, electrophoretic mobility, and effective charge of the first analyte; and
- the one or more physical properties of the second analyte represented by the second analyte property factor evidences one or more of a size, diffusion coefficient, electrophoretic mobility, and effective charge of the second analyte.

12. The precise sensing method of at least one of claims 8 to 11, wherein the one or more physical properties of the solid-state nanopore represented by the pore property factor evidences one or more of the size, shape, surface charge density, and surface roughness of the solid-state nanopore.

13. The precise sensing method of at least one of claims 8 to 12, wherein the first and second capture rates are further each a function of an operating factor that represents one or more operating conditions of the solid-state nanopore.

14. The precise sensing method of claim 13, wherein the ratio of the first capture rate to the second capture rate cancels any effect of the operating conditions of the solid-state nanopore on the concentration determinations.

**15.** The precise sensing method of at least one of claims 8 to 14, wherein the first and second capture rates are measured using a method selected from the group consisting of conductance blockage, passage time, electric charge deficit, number of distinct sublevels, event shape, event frequency spectrum, and combinations thereof.

**16.** The precise sensing method of at least one of claims 8 to 15, wherein at least one of the first analyte and the second analyte includes a bound proxy label.

**17.** The precise sensing method of claim 16, wherein the proxy label is cleaved from the at least one of the target analyte and the control analyte prior to detection.

**18.** A precise sensing method for measuring an unknown concentration using a solid-state nanopore that simultaneously translocates a first analyte and a second analyte, wherein the first analyte has an unknown concentration and the second analyte has a known concentration, the sensing method comprising:

- receiving a current signal from the solid-state nanopore over a predetermined time period and using the current signal to determine first and second population counts, wherein the first population count is a function of a single-molecule count of the first analyte and the second population count is a function of a single-molecule count of the second analyte; and
- creating a ratio of the first population count to the second population count and determining the unknown concentration using the ratio, wherein the first concentration is determined by multiplying the second concentration by the population count ratio.

110
100
110
r*
106
102
104

FIG. 1

800
600
400
200
0
Event Count
d=10 nm
d=6 nm
0
50
100
150
200
250
Time (s)
FIG. 2A
210
5
4
3
2
1
R (Hz/nM)
L*
$10^3$
$10^4$
DNA Length (bp)
FIG. 2B

d=7nm, 10 kbp
d=2.5 nm, 50 bp
Relative Capture Rate
Voltage (mV)
1.0
0.5
0.0
0
200
400
600
800

FIG. 2C

$N(t)=Jt$
Event Count / 1000
2.0
1.5
1.0
0.5
0.0
0
100
200
300
Time (s)
FIG. 3A
$S(t)=e^{-Jt}$
Survival Probability
$10^0$
$10^{-1}$
$10^{-2}$
$10^{-3}$
$10^{-4}$
0.0
0.5
1.0
Inter-Event Time (s)
FIG. 3B
Count
200
150
100
50
0
-4
-3
-2
-1
0
$\log_{10}$(Inter-Event Time (s))
FIG. 3C

Open Pore Current (nA)
11.4
11.2
11.0
$\frac{\Delta I}{I} \sim 3\%$
0
400
800
1200
Time (s)
FIG. 4A
Event Count / 1000
3
2
1
0
$\frac{\Delta J}{J} \sim 30\%$
0
400
800
1200
Time (s)
FIG. 4B

3000
2500
2000
0
5000
10000
Time (s)
Event Count / 500s
12.4
12.0
11.6
11.2
10.8
Open Pore Current (nA)

FIG. 5

Pore 1 10kbp
Pore 2 10kbp
Pore 3 20kbp
R (Hz/nM)
Voltage (mV)
0
1
2
3
4
5
100
200
300
400

FIG. 6

710
10
5
4
3
2
1
R (Hz/nM)
L*
$10^3$
$10^4$
DNA length (bp)
720
10
8
6
4
2
0
Count
CV=0.29
1
2
3
4
5
6
7
R (Hz / nM)

FIG. 7A

FIG. 7B

800
812
812
810
810
802
806
804

FIG. 8

Normalized Count / 500s
0.056
0.054
0.052
0.050
0.048
0.046
0.044
250bp
1500bp
0
2000
4000
6000
8000
10000
Time (s)

FIG. 9A

$R_2$ (Hz/nM)
6
4
2
0
2
3
4
$R_1$ (Hz/nM)

FIG. 9B

1000
Start
1002
Receiving Current Signal
1006
Identifying Sets of Distinguishable Electrical Signatures
1010
Determining First Capture Rate
Determining Second Capture Rate
1014
1018
Receiving Known Values
1022
Creating a Ratio of the First Capture Rate to the Second Capture Rate
1026
Using the Ratio to Determine an Unknown Concentration
End

FIG. 10

$10^1$
$10^0$
$J_T/J_{IC}$
$10^0$
$10^1$
$c_T/c_{IC}$
2
1
0
$r_J$
2
4
6
8
10
$L_{IC}/L_T$


FIG. 11A

FIG. 11B

Count
9
6
3
0
0
1
2
$r_J$


FIG. 11C

$10^1$
$10^0$
$N_T/N_{IC}$
$10^0$
$10^1$
$c_T/c_{IC}$
FIG. 12A
2
1
0
$r_N$
2
4
6
8
10
$L_{IC}/L_T$
FIG. 12B

12
8
4
0
Count
0
1
2
$r_N$

FIG. 12C

# EUROPEAN SEARCH REPORT

Application Number
EP 19 17 8617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/092541 A1 (DRNDIC MARIJA [US] ET AL) 18 April 2013 (2013-04-18)<br>* paragraph [0043] *<br>* paragraph [0220] *<br>* paragraph [0226] *<br>* figure 21C *<br>----- | 1-18 | INV.<br>G01N33/487 |
| A | WO 2018/081178 A1 (TWO PORE GUYS INC [US]) 3 May 2018 (2018-05-03)<br>* paragraph [0008] - paragraph [0018] *<br>----- | 1-18 | |
| | | | TECHNICAL FIELDS SEARCHED (IPC) |
| | | | G01N |

The present search report has been drawn up for all claims

1

| Place of search | Date of completion of the search | Examiner |
|---|---|---|
| The Hague | 24 January 2020 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 17 8617

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2020

| Patent document cited in search report | Publication date | Patent family member(s) | Publication date |
|---|---|---|---|
| US 2013092541 A1 | 18-04-2013 | NONE | |
| WO 2018081178 A1 | 03-05-2018 | AU 2017348009 A1 | 15-11-2018 |
| | | CA 3021676 A1 | 03-05-2018 |
| | | CN 109564185 A | 02-04-2019 |
| | | EP 3440456 A1 | 13-02-2019 |
| | | JP 2019517664 A | 24-06-2019 |
| | | KR 20190003720 A | 09-01-2019 |
| | | WO 2018081178 A1 | 03-05-2018 |

EPO FORM P0459

For more details about this annex : see Official Journal of the European Patent Office, No. 12/82